# EUROPEAN PATENT APPLICATION

(11) **EP 2 338 808 A1**
(43) Date of publication of application: **29.06.2011**
(21) Application number: 11157736.7
(22) Date of filing: 21.02.2005
(51) Int. Cl.: B65D 88/16, A61L 2/26, A61L 2/20, A22B 7/00

(54) **Use of a flexible container**

(30) Priority: 21.02.2004 BE 200400099; 01.09.2004 BE 200400424
(62) Divisional of application: 05714376.0
(71) Applicant: Alami, Abdelkader, 9160 Lokeren (BE)
(72) Inventor: Alami, Abdelkader, 9160 Lokeren (BE)
(74) Representative: Michalík, Andrej

(57) **Abstract**

The present invention provides use of a flexible container of the type FIBC for disposal of infected non-human animals, wherein the flexible container allows for regulating the gaseous environment surrounding the animals deposited there within.

## Description

### FIELD OF THE INVENTION

The present invention relates to flexible containers for transportation and storage of solid materials and goods, which are known in the art as "flexible intermediate bulk containers" or FIBC.

### BACKGROUND OF THE INVENTION

FIBC, further known in commerce as "bulk bags" or "big bags", are commonly utilized to transport and store various kinds of powdered, granular, particulate, pelletised or briquetted solid materials, such as industrial materials, food products and pharmaceutical grade materials.

While containers of this type may greatly vary in their characteristics, an exemplary FIBC has a substantially rectangular body constructed by sewing together rectangular panels along their adjacent edges to provide for side walls, base, and cover. The cover contains a filling opening which is usually fitted with a filling spout and the base often contains a discharge opening fitted with a discharge spout. The spouts are closable, such as by tying with a rope, thus sealing off the openings. The FIBC contains one or more lifting means, e.g., lifting loops, attached to the side walls or to the side edges to facilitate handling of the FIBC by a forklift hoist means.

A typical FIBC is made of a single skin of woven fabric. The fabric is usually a weave of polyolefin, e.g., polypropylene, and may or may not be laminated with a similar polyolefin on one or both of its sides. If such lamination is applied, the fabric will be non-porous, and thus particularly suited for the transport of, e.g., finely grinded or highly hygroscopic materials, while fabric without lamination will typically be porous. An FIBC may be further provided with an inner liner, which serves to accommodate the transported materials, while the outer fabric body offers structural support. Such liner may be either tubular or fitted to the shape of the FIBC and is attached to the outer body, e.g., by tying, gluing or sewing. The liner is usually made of non-woven fabric, e.g., polyethylene, as is therefore not porous.

Some materials shipped using FIBC, such as food products, may be susceptible to deterioration during transport. Moreover, materials which can sustain microbial growth, such as viral, bacterial or fungal growth, can become contaminated upon loading or during transport, and this may not only damage the cargo, but also spread microbial species from areas where they are prevalent to other areas. If such microbial species are pathogenic, this may lead to spreading of diseases. Similarly, some materials can support survival of insects, pests or their germs, which may be introduced into the cargo upon loading or enter during transport. This too may cause damage to the shipped materials and lead to spreading of such insects or pests. Additionally, known FIBC are not suitable for transporting materials which may be harmful for the environment, e.g., biologically hazardous materials comprising infectious agents, because known FIBC cannot efficiently prevent release of such agents into environment.

Accordingly, the aim of the present invention is to overcome these limitations of currently existing containers of the type FIBC and provide a greatly improved container which may be particularly useful for novel applications, such as for the transport of sensitive goods or biologically hazardous materials. The solution provided by the invention and its advantages are explained in the following.

### SUMMARY OF THE INVENTION

In one aspect, the present invention provides an improved container of the type FIBC which allows for regulating the gaseous environment which surrounds the load deposited within the container. To this aim, the present container comprises an inner space for receiving a load, said inner space being enclosed by walls made of a material that is impermeable to liquids and gases. The walls comprise at least one filling opening through which the load can be introduced into the inner space of the container. The filling opening can be hermetically closed off, so as to become impervious to liquids and gases. The walls comprise one or more valved apertures, through which gases can be drawn from or introduced into the inner space of the container. The valved apertures allow for passage of gases in open position, but form a barrier for gas flow in closed position.

Accordingly, the invention provides a flexible container of the type FIBC comprising:
- an outer body, and
- an inner space,
characterized in that walls of the inner space comprise:
- a filling opening which can be hermetically closed by a closing means,
- at least one valved aperture.

Controlling the gaseous environment surrounding the load in the presently disclosed container offers distinct advantages. For example, air can be drawn out of the inner space of the container encompassing the load, thus generating negative pressure. An inert gas may then be introduced into the container to surround the deposited load. Maintaining the load under negative pressure or under atmosphere of an inert gas during shipment (i) may prevent deterioration of sensitive goods, (ii) may counteract the survival and growth of microbial agents, such as viruses, bacteria or fungi within the load, and (iii) may be detrimental to survival of any insects or pests present in the load or to development of their germs. Furthermore, in the present container the load is separated from external environment by a barrier impermeable to liquids and gases and therefore the transported materials or goods are unlikely to become contaminated by biological agents, such as viruses, bacteria fungi, insects, pests, or by other contaminants, such as dirt or dust, during shipment. Nevertheless, if any suspicion arises that the load may have become contaminated by biological agents, the present container allows for the entire contents to be treated with a disinfecting gas prior to unloading. Importantly, the container not only protects the transported goods or materials, but in doing so it also prevents the escape of any potentially pathogenic microbial agents and harmful insects or pests from the interior and spreading of such agents between different places and countries. The inert gas atmosphere may also be beneficial for shipment of hygroscopic or corrosive materials.

In a particularly useful application, the present container may be employed to protect the external environment from the shipped materials. For example, the container may be used to transport biologically hazardous materials. The liquid and gas impermeable walls enclosing the materials will prevent any of it to be released into the environment. Moreover, if the contents is kept under negative pressure, then any accidental leaks will suck air or liquids into the container rather than releasing any material. Furthermore, after loading, the material within the container may be treated with disinfecting gas before transport. In addition, the outer surface of the container may be thoroughly disinfected before transport. Exemplary biologically hazardous materials for transport with the container of the present invention may be cadavers of animals which have to be disposed of in order to contain an outbreak of a particular infectious animal disease. It is vital that transport of these cadavers does not further contribute to spreading of the infectious agent. Exemplary diseases may include, among others, bird plague in poultry (chicken, turkey, duck, etc.), bird flu in poultry, BSE in cattle, pig plague in pig, foot and mouth disease in cloven-hoofed livestock. Another exemplary biologically hazardous materials which can be safely disposed of using the present container are the excrements of such infected animals. An added advantage of using the present container for this purpose is that, provided the entire container is made of plastic, the filled container may be directly incinerated or buried without the need to first release the hazardous contents. Such container is disposable, unlike rigid metal containers which must be extensively disinfected each time they are used for such application.

Accordingly, in an aspect, the present invention provides a method for transporting load using the present container, comprising the steps of:
(i.) introducing the load into the inner space via the filling opening,
(ii.) hermetically closing the filling opening using the closing means,
(iii.) drawing air out of the inner space,
(iv.) optionally introducing a gas into the inner space, and
(v.) transporting the container.

### BRIEF DESCRIPTION OF THE FIGURES

**Figure 1** illustrates a front view of an embodiment of the container according to the invention, attached to a trolley.
**Figure 2** illustrates a top view of the container of Figure 1 attached to a trolley.
**Figure 3** illustrates a front view of another embodiment of the container according to the invention.
**Figure 4** illustrates a top view of the container as in Figure 3.

### DETAILED DESCRIPTION OF THE INVENTION

The present invention provides a modified container of the type FIBC which allows for regulating the gaseous environment surrounding the load deposited within the container. Several characteristics of the container are commonly found in existing FIBC and embodiments of the present container defined by variations in these characteristics will be evident to a skilled person.

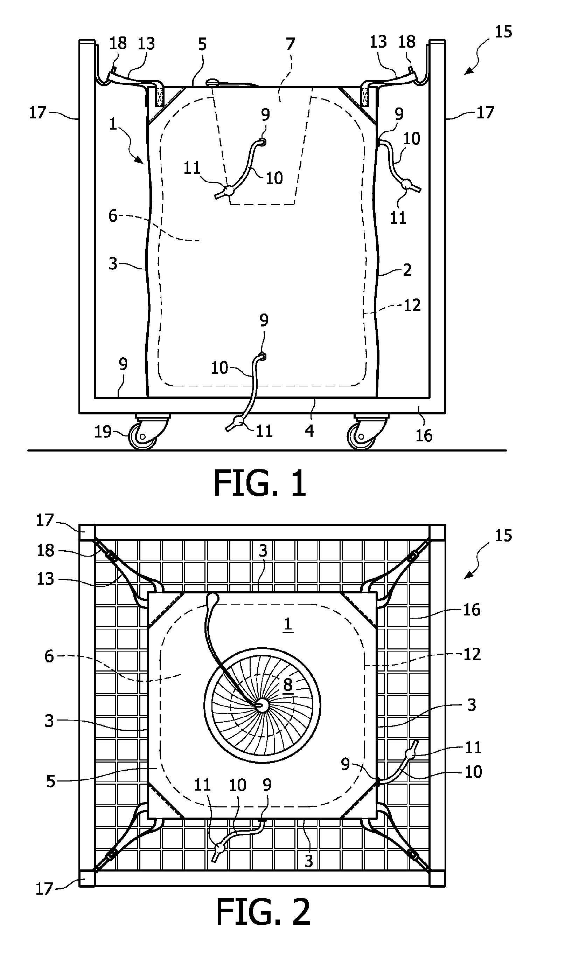

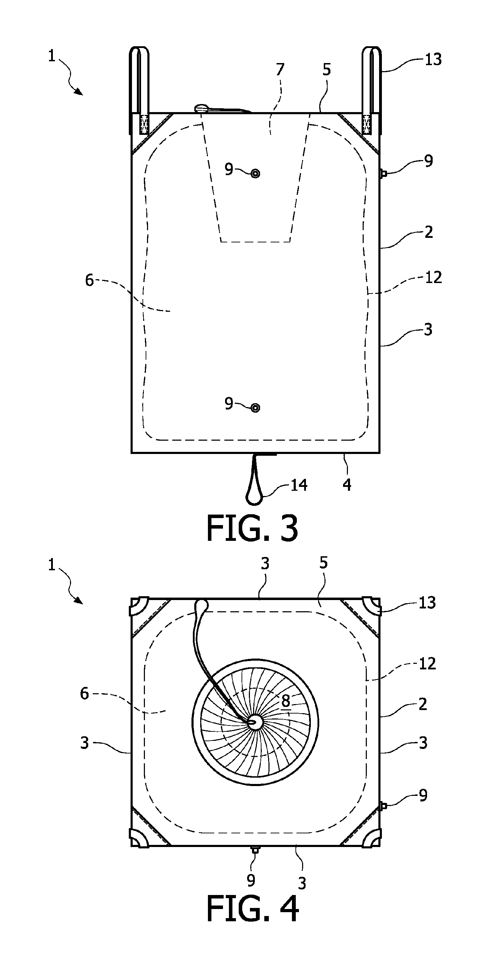

The present invention is further explained with reference to Figures 1 to 4, which illustrate two exemplary, but non-limiting preferred embodiments of the invention.

The invention provides a flexible container of the type FIBC comprising:
- an outer body (2), and
- an inner space (6),
characterized in that walls of the inner space (6) comprise:
- a filling opening (7) which can be hermetically closed by a closing means (8),
- at least one valved aperture (9).

The container 1 comprises an outer body 2 which may assume any shape used for FIBC, e.g., circular, cube, rectangular or octagonal shape. In an embodiment, the outer body 2 will preferably have a substantially rectangular shape when the container 1 is prepared for receiving load or is filled. Rectangular containers are stable when filled, they can be stacked vertically, and they ideally fill most of the available space in transporting vehicles.

The outer body of the container 1 may be constructed by known methods, such as by joining together separate panels along their adjacent edges, thus providing for side walls 3, base 4, and a top wall 5.

The outer body 2 is made of flexible, lightweight, and physically strong material. This ensures that an empty container can be readily folded into a compact shape without rupturing, that an empty container is relatively light and can be carried and manipulated by a worker without machinery, and that the body can withstand considerable pressures from the load without tearing or large distortions in the container's shape.

Materials useful for body structures of FIBC are known to a skilled person and may be employed for the outer body 2 of the embodiments of the present container 1. Such materials may include plastic materials, and in particular extruded or woven plastic materials. Woven plastic fabric may advantageously provide for excellent physical strength, which is required for the outer body 2 when the container 1 is to transport bulk materials. Typically, woven fabric is prepared by weaving of plastic fibers (i.e.,circular cross section) or strips (i.e.,flat cross section). Strips may preferred as it requires less strips than fibers to weave a fabric of a particular surface and the weaving step may therefore be less demanding. The plastic material used for woven fabric is often polyolefin, typically polyethylene or polypropylene, and preferably polypropylene. Typically, the outer body 2 is made of a single skin of such woven fabric. If the container is intended for high density materials, the outer body 2 may be made of two or more layers of such woven fabric to provide for extra strength.

The outer body 2 of the container 1 encompasses an inner space 6 which serves to receive the load. The present invention allows for controlling the gaseous atmosphere within this inner space 6. To this aim, the walls defining the inner space 6 are made of a material which is impervious, i.e., impermeable, to liquids and gases. In particular, the material is impermeable to water and aqueous liquids (i.e.,watertight) and to air, the gaseous components of air (i.e., airtight), and other gases, such as, e.g., nitrogen, carbon dioxide, or chlorine. Impermeable or impervious means that the material provides a barrier through which such substances cannot pass. Hence, the walls enclosing the inner space 6 of the container 1 can prevent liquids and gases from the enviroment to enter the inner space 6, and liquids and gases from the inner space 6 to be released into the environment. The container 1 allows to control any such exchange of liquids and gases between the inner space 6 and the environment.

The volume of the inner space 6 for accomodating the load can be any, e.g., more than 5 liters, or more than 20 liters. Typically, the volume of the container may be above 50 liters and may range up to 3000 liters or more. The dimensions of the container 1 will be chosen accordingly to provide for a desired volume of the inner space. This can be done by a skilled person.

In embodiment, the walls defining the inner space 6 may be the walls 3, 4, 5 of the outer body 2 of the container. In such case, the outer body 2 is made of a non-porous material. For example, the outer body may be made of extruded plastic sheet. Alternatively, the outer body 2 may be made of laminated woven fabric. While woven fabric is usually porous, it can be made non-porous and impermeable to liquids and gases by coating (i.e., laminating) with a film of extruded material, such as a film of polyethylene, polypropylene or PVC, which is applied to one or both surfaces of the fabric. Methods for coating woven fabric are known in the art. If the walls 3, 4, 5 of the outer body 2 define the inner space 6, then the walls must be joined without introducing unwanted openings. For example, if the walls are joined by sewing, which may introduce puncture holes, the resulting seams must be sealed, e.g., by a seal tape. Alternatively, if the material of the outer body 2 allows, the walls 3, 4, 5 may be joined by welding. The advantage of this embodiment is its simple desing, as the walls 3, 4, 5 fulfill both the function of the outer body 2 to provide for structural strength supporting the bulk material and the function of hermetically enclosing the inner space 6.

Alternatively, in an embodiment the container is provided with a flexible inner liner 12. Here, the walls of the liner 12 define the inner space 6 which receives the load. The inner surface of the liner 12 contacts the loaded material, while the outer surface of the liner 12 contacts and leans against the inner surface of the walls 3, 4, 5 of the outer body 2 of the container 1, which offers the structural support. In this embodiment the functions may be divided - the inner liner 12 provides for the possibility of hermetically enclosing the load, while the outer body 2 provides for the structural strength needed to carry bulk material. This embodiment may be preferred as it may be easier in manufacture of the container 1 to provide for an inner liner 12 which is impermeable for liquids and gases than to ensure that the outer body 2 hermetically isolates the load. For example, the outer body 2 is usually made of woven fabric, which is porous and must therefore be laminated to become impermeable. On the other hand, the inner liner 12 may be made of non-porous, impermeable material, e.g., a sheet of extruded plastic. Accordingly, in an embodiment, the inner liner 12 is made of a material impermeable to liquids and gases. Also, the panels of the outer body 2 are typically joined by sewing, and elements such as lifting means may also be attached to the outer body 2 by sewing. Sewing provides for the needed strength of the seams, but introduces puncture holes and measures must be taken to seal the latter if the outer body 2 should become impenetrable to liquids and gases. On the other hand, the sheets of the inner liner 12 may be joined hermetically by, e.g., gluing or heat sealing.

An inner liner 12 may have a simple tubular structure or it may by fitted to the shape of the outer body 2 of the container. A liner may be attached to the outer body 2, by, e.g., tying, gluing, or sewing. Attachment which does not introduce any puncture holes (e.g., gluing) may be preferred. Liners are commonly employed in commercial FIBC and are known to a skilled person.

The liner 12 according to the present invention will be made of a material impermeable to liquids and gases. For example, in an embodiment, the inner liner 12 is typically made of a sheet of extruded (i.e., not woven) plastic material, e.g., polyethylene, polypropylene, nylon or metallized plastic, which are non-porous and impermeable to liquids and gases. The thickness, density and strength of said sheet will depend on the nature of the material to be contained within the container. For example, if the load is likely to have sharp portions (e.g., animal cadavers having sharp claw, teeth, beaks, etc.), the material of the inner liner 12 may be thicker to prevent such sharp portions from causing cuts and unwanted openings in the liner 12. In an illustrative example, if the liner is made of polyethylene, this may be chosen from, e.g., high density polyethylene, high density and high molecular weight polyethylene, high density and ultrahigh molecular weight polyethylene, medium density polyethylene, low density polyethylene, linear low density polyethylene, or very low density polyethylene. Materials used for production of liners are known to a skilled person.

In a preferred embodiment, the liner 12 may be made of metallized plastic sheet. For example, such sheet may comprise at least one layer of a metal foil attached to at least one layer of an extruded plastic sheet. In an embodiment, the metal foil may be provided on the outer surface of the liner 12 and the plastic sheet on its inner surface. In an example, said metal foil may be sandwiched between extruded plastic sheets. In an embodiment, said metal foil may be an aluminium foil. In another embodiment, the at least one plastic sheet may be polyethylene or polypropylene.

The present container further comprises at least one filling opening 7 through which materials can be loaded into the inner space 6 of the container 1. Hence, such filling opening 7 is comprised in the walls defining the inner space 6, i.e., depending on the container's design, the filling opening 7 is in the walls of the outer body 2, or when the container is fitted with a liner 12, in the walls of the liner 12. Where the container comprises a liner 12, the filling opening 7 in the liner 12 will be aligned with a corresponding opening in the outer body 2, through which the filling opening 7 of the liner 12 will be accessible. The walls of the liner 12 may protrude through this opening in the outer body 2 to form a "skirt" encircling the opening 7. The outer body 2 and/or the liner 12 may be provided with a filling spout encircling the respective openings, to facilitate loading, e.g. by a hopper. In a preferred embodiment, the filling opening 7 may be placed on the top side 5 of the container, which can facilitate gravity loading, e.g., from a hopper, or may be placed on one of the side walls 3, which may be advantageous when the container is loaded manually. The container may comprise one or more filling openings 7.

The container 1 further comprises a closing means 8 for hermetically sealing the filling opening 7 after the container has been loaded, such that the filling opening 7 (and consequently the inner space 6) becomes impervious to liquids and gases. The closing means 8 will be provided so as to hermetically seal the filling opening 7 in the walls that enclose the inner space 6, i.e., depending on the container's design, the filling opening 7 in the walls of the outer body 2, or when the container is fitted with a liner 12, in the walls of the liner 12. Hence, when the closing means is closed or in a closed position, it provides for hermetically closing or sealing of the filling opening 7. In an embodiment, the closing means 8 is any known in the art to provide for hermetic closure, such as e.g., a chemical seal, for example one having a lens-like closure. In different embodiments, the closing means 8 may preferably be an integral element of the container, or may only be provided after the container has been loaded. Moreover, if the material of the liner 12 allows, the hermetic closing of the liner 12 may be achieved by heat sealing. If the load is to be discharged from the container after transport, the closing means 8 will preferably provide for reversible sealing. If the load is not to be discharged (e.g., when the container including the load is to be incinerated or otherwise disposed of), the closing means 8 may provide for irreversible sealing.

If the load of the container is to be discharged after transfer, this can be done through the filling opening 7. Alternatively, in an embodiment the container comprises at least one discharge opening (e.g., on the base 4 side of the container) which may be used for discharging. Such discharge opening may have a similar structure as the filling opening 7 comprising a closing means 8.

The container 1 further comprises one or more valved apertures 9 in the walls defining the inner space 6, i.e., depending on the container's design, the valved apertures 9 may be provided in the walls of the outer body 2, or when the container is fitted with a liner 12, in the walls of the liner 12. Where the valved apertures 9 are provided in the walls of the internal liner 12, the outer body 2 will contain corresponding openings to make such valved apertures 9 accessible from the outside. These valved apertures 9 provide a means of controlling the gaseous atmosphere within the inner space 6 of the container 1. The valves controlling the valved apertures 9 may be any known in the art. The valved apertures 9 can be reversibly opened (i.e., set in open position) to allow for passage of gases into and/or out of the inner space 6 of the container or closed (i.e., set in closed position) to form a physical barrier which completely blocks the passage of gases through the aperture. Hence, the valved apertures 9 provide a means for controlled passage of gases into and/or out of the inner space 6.

In an embodiment, the valved apertures 9 are provided with an external tubing 10, which facilitates manipulation. For example, if the valved apertures are provided on a liner 12, said tubing may extend through openings in the outer body 2 and be accessible from the outside. Here, the tubing may contain a distal thickening 11, which will prevent that the tubing becomes trapped in the space between the inner liner 12 and the outer body 2 and thus ensure that the tubing will remain accessible from the outside. In an embodiment, the valves controlling the passage of gases through the valved apertures 9 may reside in the tubing 10. For example, the valves may be provided in a thickening 11 within the tubing 10. Such valves may be more easily accessible to the worker.

The valved apertures 9 may allow passage of gases in both directions or only in one direction (i.e., from exterior to interior or from interior to exterior). In an embodiment, the container may comprise different valved apertures allowing for passage of gases in different directions.

Typically, the container will comprise at least one valved aperture 9 which allows for drawing of air (or other gases) from the inner space 6. This can be achieved, e.g., by connecting a mechanical or electrical suction (or vacuum) pump to said valved aperture 9. The air drawn from the inner space 6 may be released directly to the environment or may first be passed through a suitable filter or disinfecting liquid if the inner space contains biologically hazardous material. Advantageously, in an embodiment, the valved aperture 9 for drawing of air is positioned in the upper portion of the container to prevent the material deposited in the inner space 6 from entering the aperture 9 when the filled container is in upright position.

Typically, the container will comprise at least one valved aperture 9 which allows for introducing gases into the inner space 6. This can be achieved by connecting a gas source, e.g., a container with compressed gas, to said valved aperture 9. Such gas may preferably be an inert gas, e.g., nitrogen or carbon dioxide, or a gas with disinfecting properties, e.g., chlorine.

In different embodiments, the valved aperture(s) for drawing of air from the container may be the same as the valved aperture(s) for introducing gases into the container, or may be different. Advantageously, in an embodiment, the former valved aperture(s) may be provided with a one-way valve only allowing the passage of gases in the direction from interior to exterior, while the latter valved aperture(s) may be provided with a one-way valve only allowing the passage of gases from exterior to interior.

In an embodiment, the container comprises at least 2 valved apertures (9). In an embodiment, the container comprises 3 valved apertures (9). In an embodiment, the container comprises 3 or more valved apertures (9).

In an embodiment, the valved apertures may be covered by a diaphragm permeable to gases but impervious to liquids or particulate material on the surface facing the inner space 6. Such diaphragm would protect the tubing connected to the aperture and the suction pump or other connected devices or gas sources from entering liquids or particulate matter.

The container 1 further comprises lifting means 13, which in an embodiment may advantageously be lifting loops or straps attached to the walls 3, 4, 5, or side edges of the outer body 2 in its upper part. The lifting means 13 may preferably protrude above the level of the top wall 5, so that they can be easily accessed. The lifting means 13 may be attached to the container 1 by, e.g., sewing or welding. The lifting means 13 facilitates manipulation of the container 1 by a forklift truck or crane, because the fork arms or crane hook can easily fit the within the lifting means 13, e.g., in the openings of lifting loops. The container 1 may preferably be provided with four lifting loops 13, one on each side wall 3 or on each junction between side walls 3, as illustrated in Figures 1 to 4. FIBC containers are commonly fitted with lifting means and the lifting means may be any known in the art.

The lifting means may also be used to position the present container on a trolley. Figures 1 and 2 depict a trolley 15 having a base 16 and upright members 17. The upright members 17, e.g., shafts, are provided with attachment means 18, e.g., hooks, which can hold the lifting means 13 of the container 1. The attachment means 18 are provided at such height so as to maintain the container 1 in upright position for loading. The base 4 of the container 1 will rest on the base 16 of the trolley 15, which thus helps to carry the weight of the load. The trolley 15 may advantageously comprise wheels 19 to facilitate moving of the trolley with the attached container 1 at a workplace. Such trolley may preferably be demontable.

In an embodiment, the container 1 may comprise one or more additional loop 14 to further facilitate manipulation or emptying of the container.

The following describes a preferred way of using the container of the present invention to transport load, such as any materials or goods. After the load has been introduced into the inner space 6 of the container 1 via the filling opening 7, the opening 7 is hermetically sealed using the closing means 8. Subsequently, the air present in the inner space 6 may be drawn out using a suction (i.e., vacuum) pump connected to an appropriate valved aperture 9 (in open position) located in the upper portion of the container 1. This generates negative pressure inside the inner space 6. The valved aperture 9 is then closed and the container 1 may be shipped with the materials or goods maintained under negative pressure. Alternatively, after drawing out of the air, another gas or gases may be introduced into the inner space 6 through the same valved aperture 9 or through one or more other valved apertures 9 (in open position). After introducing the gas, the valved apertures 9 are closed and the container may be shipped. A useful example is an inert gas, such as nitrogen or carbon dioxide, which would ensure that the materials or goods are shipped under an inert atmosphere, thus preventing deterioration of load and survival of biological agents. In addition, following the transport and before opening of the filling 7 or discharge opening(s) which are hermetically sealed by the closing means 8, the materials or goods can be treated within the inner space 6 using another gas. Hence, the materials or goods may be treated with specific gases chosen to attain specific aims (e.g., disinfection with a gas having disinfecting properties, such as chlorine) before unloading.

Accordingly, in an aspect, the present invention provides a method for using the present container 1 in transporting load, which comprises the steps of:
(i.) introducing the load into the inner space 6 via the filling opening 7,
(ii.) hermetically closing the filling opening 7 using the closing means 8,
(iii.) drawing air out of the inner space 6,
(iv.) optionally introducing a gas into the inner space 6, and
(v.) transporting the container 1.

In an embodiment, said gas is an inert gas. In an embodiment, said inert gas is nitrogen or carbon dioxide.

In an embodiment, the load comprises biologically hazardous material. In an embodiment, the biologically hazardous material comprises cadavers or excrements of animals infected or suspected of being infected with an infectious agent. In an embodiment, such animals comprise poultry (chicken, turkey, duck, etc.) having bird plague or bird flu, cattle having BSE, pigs having pig plague, cloven-hoofed livestock having foot and mouth disease.

Such animals may be culled in a humane way (e.g., by suffocation with carbon dioxide gas, or electrocution) before loading in the container. For example, poultry (e.g., chicken) may be let to inhale a mixture of CO₂ gas and oxygen. Oxygen causes the chicken to hyperventilate, leading to faster, i.e., more humane, suffocation (about 20-30 sec) by the added CO₂ gas than if the CO₂ gas was provided alone (about 40 sec). The chicken may be culled before being loaded into the container, or after having been deposited into the container, where they may be gassed, e.g., with a mixture of CO₂ gas and oxygen introduced through the valved apertures of the container. A useful container may have sufficient volume to accommodate, e.g., more than 100 chicken and up to 250, 300 or more chicken. A further disinfecting composition may be added to such filled container before this is hermetically closed and vacuum pumped.

Accordingly, the container may be used for disposal of infected animals, especially infected poultry, wherein the animals are culled, deposited within the inner space 6 of the container 1 via the filling opening 7, whereafter the filling opening 7 is hermetically sealed using the closing means 8, and air is drawn from the inner space using a suction pump connected to a valved aperture 9, thus creating negative pressure. The containers may then be shipped, or alternatively another gas may first be introduced into the inner space 6. Advantageously, the entire container, including the deposited animal cadavers, may be incinerated after transport, thus destroying the infectious agent.

In an embodiment, the animals may be culled (e.g., by gassing or electrocution) prior to being deposited within the container. In another embodiment, the animals may first be deposited within the container and subsequently culled within the container by introducing a gas, e.g., CO₂ gas or a mixture of CO₂ gas and oxygen, through the valved apertures 9.

Accordingly, the present invention provides subject-matter as set forth in any one and all of (i) to (xi) below:
(i) Use of a flexible container of the type FIBC for disposal of infected non-human animals, wherein the flexible container allows for regulating the gaseous environment surrounding the animals deposited there within.
(ii) Use as set forth in (i) above, wherein said animals are culled after being deposited within the container.
(iii) Use as set forth in (ii) above, wherein said animals are culled by gassing within the container.
(iv) Use as set forth in (iii) above, wherein said gassing is with CO2 gas or a mixture of CO2 gas and oxygen introduced within the container.
(v) Use as set forth in any of (i) to (iv) above, wherein the flexible container comprises an outer body (2) and an inner space (6), and the walls of the inner space (6) comprise a filling opening (7) which can be hermetically closed by a closing means (8) and at least one valved aperture (9).
(vi) Use as set forth in (v) above, wherein the walls of the inner space (6) are made of material impermeable to liquids and gases.
(vii) Use as set forth in any of (v) or (vi) above, wherein the flexible container comprises an inner liner (12) the walls of which define the inner space (6), the inner liner (12) made of material impermeable to liquids and gases, preferably polyethylene, polypropylene, nylon or metallized plastic.
(viii) Use as set forth in any of (v) to (vii) above, wherein after having been deposited into the container the animals are gassed by introducing a gas, preferably CO2 gas or a mixture of CO2 gas and oxygen, into the container through the valved aperture (9).
(ix) Use as set forth in any of (i) to (viii) above, wherein the said animals are poultry, preferably chicken.
(x) Use as set forth in any of (i) to (ix) above, wherein the flexible container has sufficient volume to accommodate more than 100, and preferably up to 250, 300 or more chicken.
(xi) Method for culling and disposal of infected poultry comprising the steps of:
   - depositing the infected poultry within a flexible container as defined in any of (i) to (x) above;
   - culling the infected poultry by gassing within the flexible container;
   - hermetically sealing the flexible container;
   - transporting the flexible container, and
   - disposing of the said flexible container.

In another aspect, the present invention provides a method for disinfecting the load within the inner space 6 comprising introducing into the inner space 6 a gas with disinfecting properties from a source connected to a valved aperture 9.

## Claims

1. Use of a flexible container of the type FIBC for disposal of infected non-human animals, wherein the flexible container allows for regulating the gaseous environment surrounding the animals deposited there within.

2. Use according to claim 1, wherein said animals are culled after being deposited within the container.

3. Use according to claim 2, wherein said animals are culled by gassing within the container.

4. Use according to claim 3, wherein said gassing is with CO₂ gas or a mixture of CO₂ gas and oxygen introduced within the container.

5. Use according to any of claims 1 to 4, wherein the flexible container comprises an outer body (2) and an inner space (6), and the walls of the inner space (6) comprise a filling opening (7) which can be hermetically closed by a closing means (8) and at least one valved aperture (9).

6. Use according to claim 5, wherein the walls of the inner space (6) are made of material impermeable to liquids and gases.

7. Use according to any of claims 5 or 6, wherein the flexible container comprises an inner liner (12) the walls of which define the inner space (6), the inner liner (12) made of material impermeable to liquids and gases, preferably polyethylene, polypropylene, nylon or metallized plastic.

8. Use according to any of claims 5 to 7, wherein after having been deposited into the container the animals are gassed by introducing a gas, preferably CO₂ gas or a mixture of CO₂ gas and oxygen, into the container through the valved aperture (9).

9. Use according to any of claims 1 to 8, wherein the said animals are poultry, preferably chicken.

10. Use according to any of claims 1 to 9, wherein the flexible container has sufficient volume to accommodate more than 100, and preferably up to 250, 300 or more chicken.

11. Method for culling and disposal of infected poultry comprising the steps of:
- depositing the infected poultry within a flexible container as defined in any of claims 1 to 10;
- culling the infected poultry by gassing within the flexible container;
- hermetically sealing the flexible container;
- transporting the flexible container, and
- disposing of the said flexible container.
